# EUROPEAN PATENT APPLICATION

(11) **EP 4 652 874 A1**
(43) Date of publication of application: **26.11.2025**
(21) Application number: 24177562.6
(22) Date of filing: 23.05.2024
(51) Int. Cl.: A24F 40/30, A24F 40/90, A24F 40/05, A61M 15/06

(54) **AEROSOL GENERATING APPARATUS**

(71) Applicant: Imperial Tobacco Limited, Bristol, BS3 2LL (GB)
(72) Inventor: The designation of the inventor has not yet been filed
(74) Representative: Haseltine Lake Kempner LLP

(57) **Abstract**

There is provided an aerosol generating apparatus (1) comprising: a first aerosol generation unit (4) configured to generate a first aerosol from a first aerosol precursor (6); a second aerosol generation unit (5) configured to generate a second aerosol from a second aerosol precursor (7); a power supply (2) arranged to provide power to the first and second aerosol generation units (4, 5); a charging port (9) arranged to receive power from an external power source so as to charge the power supply (2); a first driving circuit 300 configured, in a driving configuration, to drive the first aerosol generation unit (4) to generate the first aerosol; and a second driving circuit 301 configured, in a driving configuration, to drive the second aerosol generation unit (5) to generate the second aerosol. The first and/or second driving circuits are controllably switchable to switch to a charging configuration, and when the first or second driving circuit is in the charging configuration, said driving circuit is configured to transfer power from the charging port (9) to the power supply (2) to charge the power supply.

## Description

### FIELD

The present disclosure relates to an aerosol generating apparatus.

### BACKGROUND

A typical aerosol generating apparatus may comprise a power supply, an aerosol generating unit that is driven by the power supply, an aerosol precursor, which in use is aerosolised by the aerosol generating unit to generate an aerosol, and a delivery system for delivery of the aerosol to a user.

In some cases, the aerosol generating unit may include an ultrasonic generator e.g. a piezoelectric transducer (PET) for generating the aerosol. In use, the surface of the PET will expand and contract as it vibrates.

A PET generates aerosol by causing cavitation to occur within a liquid aerosol precursor that is provided on a surface of the PET. Cavitation refers to the phenomenon where the static pressure of a liquid reduces to below the liquid's vapour pressure, leading to the formation of small vapour filled cavities within the liquid. When the cavities are subsequently subjected to a higher pressure, the cavities collapse resulting in a shock wave that propagates through the liquid. This shock wave induces capillary waves, or ripples, in a surface distal (referred to herein as the upper surface of the liquid) from the PET that may form ligaments to expel droplets from the upper surface. More succinctly, in a thin layer of liquid, the collapsing of the cavities can induce a disturbance in the liquid that causes liquid droplets to be expelled from liquid, thereby forming an aerosol over the surface of the liquid, typically within an aerosolisation chamber.

In the context of a PET for generating the aerosol, when the surface of the PET expands, the liquid on the surface will conform to the expanded surface. When the surface of the PET subsequently contracts, the static pressure in the liquid will fall as it is effectively dragged with the surface with the decrease in static pressure being proportional to the speed of the movement of the PET surface (i.e., the frequency of the vibration). If the frequency and the amplitude of vibration of the PET is sufficiently high, cavitation will occur as a result of the contraction.

When the surface of the PET subsequently expands, the static pressure in the liquid will rise as it is effectively compressed by the surface with the increase in static pressure being proportional to the speed of the movement of the PET surface (i.e., the frequency of the vibration). Any cavities in the liquid previously formed may then implode, generating shock waves in the liquid capable of expelling droplets to form an aerosol.

In an aerosol generating apparatus using a PET, a liquid aerosol precursor is typically applied to the PET surface using a wick in fluid communication with a tank. The aerosol generated by cavitation of the liquid aerosol precursor will be drawn from the aerosolisation chamber along an aerosol flow path by suction at a mouthpiece outlet.

Aerosol generating apparatuses that use a PET for generating the aerosol present numerous challenges, including accurately driving the vibrational element and inefficiencies in the requisite circuitry.

In spite of the effort already invested in the development of aerosol generating apparatuses/systems further improvements are desirable.

### SUMMARY

In an aspect, the present disclosure provides an aerosol generating apparatus. The aerosol generating apparatus comprises a first aerosol generation unit, a second aerosol generation unit, a power supply, a charging port, a first driving circuit, and a second driving circuit. The first aerosol generation unit is configured to generate a first aerosol from a first aerosol precursor. The second aerosol generation unit is configured to generate a second aerosol from a second aerosol precursor. The power supply is arranged to provide power to the first and second aerosol generation units. The charging port is arranged to receive power from an external power source so as to charge the power supply. The first driving circuit is configured, in a driving configuration, to drive the first aerosol generation unit to generate the first aerosol. The second driving circuit is configured, in a driving configuration, to drive the second aerosol generation unit to generate the second aerosol. The first and/or second driving circuits are controllably switchable to switch to a charging configuration. When the first or second driving circuit is in the charging configuration, said driving circuit is configured to transfer power from the charging port to the power supply to charge the power supply.

In some examples, one of the first and second driving circuits may be switched to the charging configuration (e.g., from the driving configuration) in response to a determination that an external power source is connected to the charging port.

Operation of the aerosol generating apparatuses described herein may be maintained whilst the apparatus is charged without requiring the mechanical and electrical burden of need a bespoke charging circuit. In other words, the aerosol generating apparatuses as described herein may be beneficial because they do not require the costly/complex addition of bespoke charging circuitry which may be difficult to achieve within the dimensional constraints of the aerosol generating apparatus. Specifically, it allows for an aerosol generating apparatus to be provided that can simultaneously be operated and charged without requiring an unnecessarily large/bulky apparatus to accommodate the required circuitry.

The first driving circuit may be arranged to electrically (and switchably) connect the power supply to the first aerosol generation unit, the power supply to the charging port, and the first aerosol generation unit to the charging port.

Similarly, the second driving circuit may be arranged to electrically (and switchably) connect the power supply to the second aerosol generation unit, the power supply to the charging port, and the second aerosol generation unit to the charging port.

In some examples, the first and/or second driving circuits may be further controllably switchable to switch to an on-charge driving configuration. The switch to the on-charge driving configuration may be in response to a determination that the charging port is receiving power from an external power source such that, in response to said determination, one of the first and second driving circuits may be switched to the charging configuration, and the other of the first and second driving circuits may be switched to the on-charge driving configuration. When the first or second driving circuit is in the on-charge driving configuration, said driving circuit may be configured to transfer power from the charging port to the corresponding aerosol generation unit so as to generate a corresponding aerosol.

In this way, power for operating the aerosol generating apparatus may be drawn from the external power source such that there is no attempt to drain the power supply of the aerosol generating apparatus while it is being charged.

For example, an aerosol generating apparatus may be used with both the first and second driving circuits in the driving configuration to generate first and second aerosols using the first and second aerosol generation units respectively. In response to an external power source being connected to the charging port of the aerosol generating apparatus, the first driving circuit may be switched from the driving configuration to the charging configuration, and the second driving circuit may (simultaneously or consequently) be switched from the driving configuration to the on-charge driving configuration such that the aerosol generating apparatus is still operable to generate the second aerosol while the power supply is charged. Conversely, in response to an external power source being connected to the charging port of the aerosol generating apparatus, the second driving circuit may be switched from the driving configuration to the charging configuration, and the first driving circuit may (simultaneously or consequently) be switched from the driving configuration to the on-charge driving configuration such that the aerosol-generating apparatus is still operable to generate the first aerosol while the power supply is charged.

The first and/or second driving circuits may, in some examples, be switchable between any of the driving configuration, charging configuration and on-charge driving configuration.

In some examples, the first aerosol generation unit may be a first piezoelectric transducer and/or the second aerosol generation unit may be a second piezoelectric transducer.

In some examples, the first driving circuit may include a first switch for facilitating the application of current at a first driving frequency through the first piezoelectric transducer so as to induce vibrations in the first piezoelectric transducer at the first driving frequency. Additionally or alternatively, in some examples, the second driving circuit may include a second switch for facilitating the application of current at a second driving frequency through the second piezoelectric transducer so as to induce vibrations in the second piezoelectric transducer at the second driving frequency.

In other words, in examples where the first aerosol generation unit is a first piezoelectric transducer, the first piezoelectric transducer may be controllably driven at the first driving frequency through actuation of the first switch of the first driving circuit. Similarly, in examples where the second aerosol generation unit is a second piezoelectric transducer, the second piezoelectric transducer may be controllably driven at the second driving frequency through actuation of the second switch of the second driving circuit.

Each of the first and second piezoelectric transducers (when present) may be configured to induce cavitation in a received aerosol precursor received on the surface of the respective piezoelectric transducer to thereby generate an aerosol from said received aerosol precursor.

In some examples, the first and second driving frequencies may be a common driving frequency. In such examples, it may be possible to embody the first and second switch as a single switch to actuate the delivery of current through the first and second driving circuits synchronously.

In some examples, the current respectively applied through the first and/or second piezoelectric transducer may be a direct current such that the corresponding piezoelectric transducer is driven with a current having a single polarity.

Upon application of a current in a first polarity, opposite faces of the piezoelectric crystal of a piezoelectric transducer respond by expanding, or bulging, outwards to define respective convex surfaces. Conversely, upon application of current in a second polarity opposite to the first polarity, the opposite faces of the piezoelectric crystal of the transducer respond by contracting, or drawing, inwards to define respective concave surfaces. In the context of an aerosol-generating apparatus, driving the piezoelectric transducer in the first polarity may ensure that physical contact between the transducer and the received aerosol precursor can be maintained. Maintaining this physical contact may improve the power efficiency of the inducement of cavitation in the aerosol precursor, and therefore may improve the efficiency of the generation of the aerosol. To this end, the current driving one or both of the first and second piezoelectric transducers may be a direct current to ensure driving of the piezoelectric transducer is carried out in a single polarity.

In some examples, the first switch may be a MOSFET switch. Additionally or alternatively, in some examples, the second switch may be a MOSFET switch. In some examples, the first and second switches may be driven by a common MOSFET power source. Alternatively, in some examples, the first and second switches may be driven by respectively different MOSFET power sources.

The piezoelectric transducers that may be used in the aerosol generating apparatuses described herein may be capable of vibrating at ultrasonic frequencies, e.g., at frequencies of 100 kHz or above, 500 kHz or above, 1 MHz or more, 2 MHz or more, 5 MHz or more, or 10 MHz or more.

To drive the first and/or second piezoelectric transducer at such high frequencies, requires a similarly fast actuation of the first and/or second switches. Such rapid actuation of switches may be readily achievable using MOSFET switches.

In some examples, the first switch may be connected to a first oscillator or first clock for actuating the first switch at the first driving frequency. Additionally or alternatively, in some examples, the second switch may be connected to a second oscillator or second clock for actuating the second switch at the second driving frequency. In some examples, the first and second oscillator/clock may be a common oscillator/clock. Alternatively, in some examples, the first and second oscillator/clock may be respectively different oscillators/clocks. For example, the first switch may be connected to an oscillator and the second switch may be connected to a clock, or vice versa; or the first and second switch may be respectively connected to first and second oscillators, or to first and second clocks.

In some examples, the power supply may be a rechargeable battery.

In some examples, one or both of the first and second aerosol precursors may be a liquid aerosol precursor. In other words, the first and/or second aerosol precursor may be in liquid form. Alternatively, the first and/or second aerosol precursor may be a gel aerosol precursor - i.e., the first and/or second aerosol precursor may be in gel form.

In some examples the first aerosol precursor may be different from the second aerosol precursor.

In some examples, the first aerosol precursor may include a nicotine-containing component. Additionally or alternatively, the second aerosol precursor may include a flavouring.

Each of the first and second aerosol generation unit may be optimised to generate a respective aerosol from the corresponding aerosol precursor. For example, in cases where one or both of the first and second aerosol generation units are piezoelectric transducers, the or each driving frequency at which the corresponding piezoelectric transducer is driven may be selected to optimise the generation of aerosol from the corresponding aerosol precursor.

For example, by controllably adjusting, or tuning, the driving frequency at which the or each piezoelectric transducer is driven, one or more properties of the respective aerosol generated by the corresponding piezoelectric transducer may be controllably adjusted - e.g., according to a user's preference, manufacturer/provider's recommendation, and/or regulatory requirement. Adjusting the driving frequency may be particularly suitable for adjusting an average size of droplets of the aerosol precursor entrained in the generated aerosol and/or a distribution of the size of said droplets. The inventors have observed that the frequency of the driving signal is a parameter that directly affects the average size of droplets in the aerosol generated by the aerosol generating apparatuses described herein. In particular, when driving the piezoelectric transducer with a driving signal having a relatively higher driving frequency, a relatively smaller average droplet size is observed. This observation is consistent with the physical mechanism for cavitation described in Kooij et al. Sci. Rep., 9, 6128 (2019), the entirety of which is incorporated herein by reference.

For example, it may be beneficial for an aerosol generated from an aerosol precursor that includes a nicotine-containing component to have droplets that are sized so as to be deposited in the lungs when the aerosol is inhaled by a user of the aerosol generating apparatus. Deposition of the aerosol in the lungs may ensure that active ingredients (e.g., nicotine) within the aerosol droplets may be more efficiently absorbed into the user's bloodstream, thereby enhancing the user's experience of using the aerosol generating apparatuses described herein. For example, the average droplet size of the aerosol generated from an aerosol precursor that includes a nicotine-containing component may be less than 2 µm.

Meanwhile, it may be beneficial for an aerosol generated from an aerosol precursor that includes a flavouring to have droplets that are sized so as to be deposited in the mouth when the aerosol is inhaled by a user of the aerosol generating apparatus. Deposition of the aerosol in the mouth may ensure that the user is able to experience a taste sensation associated with the deposition of droplets on the tongue of the user when they inhale the aerosol generated by the aerosol generating apparatuses described herein. For example, the average droplet size of the aerosol generated from an aerosol precursor that includes a flavouring may be approximately 5 µm (e.g., between 4 µm and 6 µm).

In another aspect, the present disclosure provides a method of controlling the aerosol generating apparatuses described herein. The method comprises: determining whether the charging port is receiving power from an external power source; and, in response to a determination that the charging port is receiving power from an external power source, transmitting a switching signal to the first driving circuit or the second driving circuit to cause said driving circuit to switch from the driving configuration to the charging configuration.

In this way, one of the first and second driving circuits may continue in the driving configuration such that simultaneous operation and charging of the aerosol generating apparatus is achieved.

In some examples, the method may further comprise: receiving input indicative of a user selection of which of the first and second driving circuits to switch to the charging configuration; and transmitting the switching signal to the selected driving circuit.

In this way, the user may continue which of the first and second aerosols they wish to continue receiving during user of the aerosol generating apparatus, while the apparatus is on-charge. For example, in cases where the first aerosol includes a nicotine-containing component and the second aerosol includes a flavouring, the user can select which of flavour and nicotine they wish to inhale during an on-charge operation of the aerosol generating apparatus.

In some examples, the user may be able to provide input to change which of the first and second driving circuits is in an on-charge configuration, and which is in a driving (e.g., in an on-charge driving) configuration such that the user is not constrained to only receive the first aerosol or only receive the second aerosol while the device is on charge, but rather is able to switch between the first and second aerosol as desired or needed.

In some examples, the user input may be received e.g., via a user interaction with an application on their personal device (e.g., a mobile phone). In such examples, the personal device may be communicatively connected to a communications interface of the aerosol generating apparatus, optionally via a network server.

In some examples, the user input may include one or more preset (or default) user preferences that the method may implement as standard, unless the user provides specific input to override the preset (default) preferences.

In some examples the manufacturer and/or provided of the aerosol generating apparatus may provide the device with a "factory" default such that user input is not necessarily required. In other words, the factory default may provide a default selection that can be overridden, either by a specific user input or by the user providing the one or more preset (default) user preferences.

In some examples, the switching signal may be a first switching signal, the first and/or second driving circuits may be controllably switchable to the on-charge driving configuration, and the method may further comprise: transmitting a second switching signal to the other of the first and second driving circuits to cause said other driving circuit to switch form the driving configuration to the on-charge driving configuration.

In other words, in some examples, in response to the determination that the charging port is receiving power from an external power source, the method may comprise: transmitting the first switching signal to the first driving circuit to cause the first driving circuit to switch to the charging configuration, and transmitting the second switching signal to the second driving circuit to cause the second driving circuit to switch to the on-charge driving configuration; or transmitting the first switching signal to the second driving circuit to cause the second driving circuit to switch to the charging configuration, and transmitting the second switching signal to the first driving circuit to cause the first driving circuit to switch to the on-charge driving configuration.

In another aspect, the present disclosure provides a computer-readable medium comprising instructions that, when executed by a computer, cause the computer to carry out the methods described herein.

In another aspect, the present disclosure provides electrical circuitry for an aerosol generating system, the electrical circuitry being arranged to perform any of the methods described herein.

In embodiments, the electrical circuitry is implemented as one or more processors, which are configured to implement the disclosed steps, e.g. as the controller. The processors may execute program code stored on electronic memory and/or may execute logic, e.g. as a logic array, gate array, structured gate array.

As will be apparent from the present disclosure, the methods described herein may be carried out, or implemented, by a computer. The computer may, for example, be a processor installed in the aerosol generating apparatus and configured to operate as a control unity of the aerosol generating apparatus. Alternatively, the computer may, for example, be a remote computer communicatively connectable to the aerosol generating apparatus via a communications interface of the aerosol generating apparatus. Alternatively, the computer may be embodied as a distributed computing environment, including for example, both a control unit installed in the aerosol generating apparatus and a remote computer that is communicatively connectable to the control unit via a communications interface of the aerosol generating apparatus.

Moreover, the acts described herein may be embodied using computer-executable instructions that can be implemented by one or more processors and/or stored on a computer-readable medium or media. The computer-executable instructions can include routines, sub-routines; programs; threads of execution, and/or the like. Still further, results of acts of the methods can be stored in a computer-readable medium, displayed on a display device, and/or the like.

The order of the operations of the methods described herein is exemplary, but the steps may be carried out in any suitable order, or simultaneously where appropriate. Additionally, steps may be added or substituted in, or individual steps may be deleted from any of the methods without departing from the scope of the subject matter described herein. Aspects of any of the examples described above may be combined with aspects of any of the other examples described to form further examples without losing the effect sought.

Various functions described herein can be implemented in hardware, software, or any combination thereof. If implemented in software, the functions can be stored on or transmitted over as one or more instructions or code on a computer-readable medium. Computer-readable media may include, for example, computer-readable storage media. Computer-readable storage media may include volatile or non-volatile, removable or non-removable media implemented in any method or technology for storage of information such as computer readable instructions, data structures, program modules or other data. A computer-readable storage media can be any available storage media that may be accessed by a computer. By way of example, and not limitation, such computer-readable storage media may comprise RAM, ROM, EEPROM, flash memory or other memory devices, CD-ROM or other optical disc storage, magnetic disc storage or other magnetic storage devices, or any other medium that can be used to carry or store desired program code in the form of instructions or data structures and that can be accessed by a computer.

Although illustrated as a local device it will be appreciated that the computing device may be located remotely and accessed via a network or other communication link (for example using a communication interface).

The term 'computer' is used herein to refer to any device with processing capability such that it can execute instructions. Those skilled in the art will realise that such processing capabilities are incorporated into many different devices and therefore the term 'computer' includes PCs, servers, mobile telephones, personal digital assistants and many other devices.

Those skilled in the art will realise that storage devices utilised to store program instructions can be distributed across a network. For example, a remote computer may store an example of the process described as software. A local or terminal computer may access the remote computer and download a part or all of the software to run the program. Alternatively, the local computer may download pieces of the software as needed or execute some software instructions at the local terminal and some at the remote computer (or computer network). Those skilled in the art will also realise that by utilising conventional techniques known to those skilled in the art that all, or a portion of the software instructions may be carried out by a dedicated circuit, such as a DSP, programmable logic array, or the like.

It will be understood that the benefits and advantages described above may relate to one embodiment or may relate to several embodiments. The embodiments are not limited to those that solve any or all the stated problems or those that have any or all of the stated benefits and advantages. Variants should be considered to be included into the scope of the invention.

The present disclosure may provide electrical circuitry and/or a computer program configured to cause an aerosol generating apparatus/system to perform any method or method step disclosed herein. A computer readable medium comprising the computer program is also disclosed.

The preceding summary is provided for purposes of summarizing some examples to provide a basic understanding of aspects of the subject matter described herein. Accordingly, the above-described features should not be construed to narrow the scope or spirit of the subject matter described herein in any way. Moreover, the above and/or proceeding examples may be combined in any suitable combination to provide further examples, except where such a combination is clearly impermissible or expressly avoided. Other features, aspects, and advantages of the subject matter described herein will become apparent from the following text and the accompanying drawings.

### BRIEF DESCRIPTION OF THE FIGURES

Aspects, features and advantages of the present disclosure will become apparent from the following description of examples in reference to the appended drawings in which like numerals denote like elements.
Fig. 1 is a block system diagram showing an example aerosol generating apparatus.
Fig. 2 is a block system diagram showing an example implementation of the apparatus of Fig. 1, where the aerosol generating apparatus is configured to generate aerosol from a liquid precursor.
Figs. 3a and 3b are schematic diagrams showing an example implementation of the apparatus of Fig. 2.
Fig. 4 is a block system diagram showing an example system for managing an aerosol generating apparatus.
Fig. 5 shows an example of a circuit for modelling the behaviour of an exemplary piezoelectric transducer.
Fig. 6 shows a portion of an exemplary driving circuit using an H-bridge.
Fig. 7 shows an example of an improved driving circuit for driving a piezoelectric transducer.
Fig. 8 is a block system diagram showing an example aerosol generating apparatus having first and second aerosol generation units.
Fig. 9 shows an example of first and second driving circuits for driving respective piezoelectric transducers.
Figs. 10a to 10c show exemplary configurations of three-way switches suitable for switching the driving circuits of Fig. 9 between charging, driving, and on-charge driving configurations.
Fig. 11 shows an exemplary method of controlling an aerosol generating apparatus.

### DETAILED DESCRIPTION OF EMBODIMENTS

Before describing several examples implementing the present disclosure, it is to be understood that the present disclosure is not limited by specific construction details or process steps set forth in the following description and accompanying drawings. Rather, it will be apparent to those skilled in the art having the benefit of the present disclosure that the systems, apparatuses and/or methods described herein could be embodied differently and/or be practiced or carried out in various alternative ways.

Unless otherwise defined herein, scientific and technical terms used in connection with the presently disclosed inventive concept(s) shall have the meanings that are commonly understood by those of ordinary skill in the art, and known techniques and procedures may be performed according to conventional methods well known in the art and as described in various general and more specific references that may be cited and discussed in the present specification.

Any patents, published patent applications, and non-patent publications mentioned in the specification are hereby incorporated by reference in their entirety.

All examples implementing the present disclosure can be made and executed without undue experimentation in light of the present disclosure. While particular examples have been described, it will be apparent to those of skill in the art that variations may be applied to the systems, apparatus, and/or methods and in the steps or in the sequence of steps of the methods described herein without departing from the concept, spirit, and scope of the inventive concept(s). All such similar substitutions and modifications apparent to those skilled in the art are deemed to be within the spirit, scope, and concept of the inventive concept(s) as defined by the appended claims.

The use of the term "a" or "an" in the claims and/or the specification may mean "one," as well as "one or more," "at least one," and "one or more than one." As such, the terms "a," "an," and "the," as well as all singular terms, include plural referents unless the context clearly indicates otherwise. Likewise, plural terms shall include the singular unless otherwise required by context.

The use of the term "or" in the present disclosure (including the claims) is used to mean an inclusive "and/or" unless explicitly indicated to refer to alternatives only or unless the alternatives are mutually exclusive. For example, a condition "A or B" is satisfied by any of the following: A is true (or present) and B is false (or not present), A is false (or not present) and B is true (or present), and both A and B are true (or present).

As used in this specification and claim(s), the words "comprising, "having," "including," or "containing" (and any forms thereof, such as "comprise" and "comprises," "have" and "has," "includes" and "include," or "contains" and "contain," respectively) are inclusive or open-ended and do not exclude additional, unrecited elements or method steps.

Unless otherwise explicitly stated as incompatible, or the physics or otherwise of the embodiments, examples, or claims prevent such a combination, the features of examples disclosed herein, and of the claims, may be integrated together in any suitable arrangement, especially ones where there is a beneficial effect in doing so. This is not limited to only any specified benefit, and instead may arise from an "ex post facto" benefit. This is to say that the combination of features is not limited by the described forms, particularly the form (e.g. numbering) of example(s), embodiment(s), or dependency of claim(s). Moreover, this also applies to the phrase "in one embodiment," "according to an embodiment," and the like, which are merely a stylistic form of wording and are not to be construed as limiting the following features to a separate embodiment to all other instances of the same or similar wording. This is to say, a reference to 'an,' 'one,' or 'some' embodiment(s) may be a reference to any one or more, and/or all embodiments, or combination(s) thereof, disclosed. Also, similarly, the reference to "the" embodiment may not be limited to the immediately preceding embodiment. Further, all references to one or more embodiments or examples are to be construed as non-limiting to the claims.

The present disclosure may be better understood in view of the following explanations, wherein the terms used that are separated by "or" may be used interchangeably:

As used herein, an "aerosol generating apparatus" (or "electronic(e)-cigarette") may be an apparatus configured to deliver an aerosol to a user for inhalation by the user. The apparatus may additionally/alternatively be referred to as a "smoking substitute apparatus", if it is intended to be used instead of a conventional combustible smoking article. As used herein a combustible "smoking article" may refer to a cigarette, cigar, pipe or other article, that produces smoke (an aerosol comprising solid particulates and gas) via heating above the thermal decomposition temperature (typically by combustion and/or pyrolysis). An aerosol generated by the apparatus may comprise an aerosol with particle sizes of 0.2 - 7 microns, 2-3 microns, or less than 10 microns, or less than 7 microns, or less than 3 microns, or less than 2 microns. This particle size may be achieved by control of one or more of: driving parameters of the ultrasonic generator; flow properties including turbulence and velocity. The generation of aerosol by the aerosol generating apparatus may be controlled by an input device. The input device may be configured to be user-activated, and may for example include or take the form of an actuator (e.g. actuation button) and/or an airflow sensor.

Each occurrence of the aerosol generating apparatus being caused to generate aerosol for a period of time (which may be variable) may be referred to as an "activation" of the aerosol generating apparatus. The aerosol generating apparatus may be arranged to allow an amount of aerosol delivered to a user to be varied per activation (as opposed to delivering a fixed dose of aerosol), e.g. by activating an aerosol generating unit of the apparatus for a variable amount of time, e.g. based on the strength/duration of a draw of a user through a flow path of the apparatus (to replicate an effect of smoking a conventional combustible smoking article).

The aerosol generating apparatus may be portable. As used herein, the term "portable" may refer to the apparatus being for use when held by a user.

As used herein, an "aerosol generating system" may be a system that includes an aerosol generating apparatus and optionally other circuitry/components associated with the function of the apparatus, e.g. one or more external devices and/or one or more external components (here "external" is intended to mean external to the aerosol generating apparatus). As used herein, an "external device" and "external component" may include one or more of a: a charging device, a mobile device (which may be connected to the aerosol generating apparatus, e.g. via a wireless or wired connection); a networked-based computer (e.g. a remote server); a cloud-based computer; any other server system.

An example aerosol generating system may be a system for managing an aerosol generating apparatus. Such a system may include, for example, a mobile device, a network server, as well as the aerosol generating apparatus.

As used herein, an "aerosol" may include a suspension of liquid droplets of precursor. An aerosol may include one or more components of the precursor.

As used herein, a "precursor" may include one or more of a: liquid; gel. The precursor may be processed by an aerosol generating unit of an aerosol generating apparatus to generate an aerosol. The precursor may include one or more of: an active component; a carrier; a flavouring. The active component may include one or more of nicotine; caffeine; a cannabidiol oil; a non-pharmaceutical formulation, e.g. a formulation which is not for treatment of a disease or physiological malfunction of the human body. The active component may be carried by the carrier, which may be a liquid, including propylene glycol and/or glycerine. The term "flavouring" may refer to a component that provides a taste and/or a smell to the user. The flavouring may include one or more of: Ethylvanillin (vanilla); menthol, Isoamyl acetate (banana oil); or other. The precursor may include a carrier; a flavouring.

As used herein, a "storage portion" may be a portion of the apparatus adapted to store the precursor. It may be implemented as fluid-holding reservoir depending on the implementation of the precursor as defined above.

As used herein, a "flow path" may refer to a path or enclosed passageway through an aerosol generating apparatus, e.g. for delivery of an aerosol to a user. The flow path may be arranged to receive aerosol from an aerosol generating unit. When referring to the flow path, upstream and downstream may be defined in respect of a direction of flow in the flow path, e.g. with an outlet being downstream of an inlet.

As used herein, a "delivery system" may be a system operative to deliver an aerosol to a user. The delivery system may include a mouthpiece and a flow path. The delivery system may be at least partly within the aerosol generating component.

As used herein, a "flow" may refer to a flow in a flow path. A flow may include aerosol generated from the precursor. The flow may include air, which may be induced into the flow path via a puff by a user.

As used herein, a "puff" (or "inhale" or "draw") by a user may refer to expansion of lungs and/or oral cavity of a user to create a pressure reduction that induces flow through the flow path.

As used herein, an "aerosol generating unit" may refer to a device configured to generate an aerosol from a precursor. The aerosol generating unit may include a unit to generate an aerosol directly from the precursor (e.g. an atomiser including an ultrasonic system). A plurality of aerosol generating units to generate a plurality of aerosols (for example, from a plurality of different aerosol precursors) may be present in an aerosol generating apparatus.

As used herein, an "ultrasonic generator" may refer to a piezoelectric transducer capable of vibrating at ultrasonic frequencies, i.e., at frequencies greater than 20kHz. In some examples, the piezoelectric transducer may be capable of vibrating at even higher frequencies, e.g., at frequencies of 100 kHz or above, 500 kHz or above, 1 MHz or more, 2 MHz or more, 5 MHz or more, or 10 MHz or more. The piezoelectric transducer may be adapted to vibrate in response to a driving signal, and in particular adapted to vibrate at the frequency of the driving signal. The driving signal may be generated, for example, using direct digital synthesis or any other suitable method.

As used herein, a "piezoelectric transducer" may refer to an ultrasonic transducer comprising a piezoelectric crystal, which generates a mechanical strain internally in response to an electric field. A rapidly changing electric field, such as an ultrasonic frequency driving signal, results in rapidly changing mechanical strain within the piezoelectric crystal causing it to vibrate. The piezoelectric transducer will have an aerosolisation surface from which the aerosol is generated. The aerosolisation surface typically faces into an aerosolisation chamber.

As used herein, an "aerosol **generating** component" may refer to a component that includes an aerosol precursor. The component may include an aerosol generating unit e.g. it may be arranged as a cartomizer. The component may include a mouthpiece. The component may include an information carrying medium. The component may include a storage portion, e.g. a reservoir or tank, for storage of the aerosol precursor.

With liquid or gel implementations of the aerosol precursor, e.g. an e-liquid, the component may be referred to as a "capsule" or a "pod" or an "e-liquid consumable". In some embodiments, the aerosol precursor component may be affixed to the device body to form the aerosol generating apparatus. In these embodiments, the reservoir/tank may be refillable.

The aerosol generating component e.g. the capsule, pod, or consumable may be for releasable coupling to a device body to form the aerosol generating apparatus.

The device body may comprise a power supply for powering the aerosol generating unit.

As used herein, an "information carrying medium" may include one or more arrangements for storage of information on any suitable medium. Examples include: a computer readable medium; a Radio Frequency Identification (RFID) transponder; codes encoding information, such as optical (e.g. a bar code or QR code) or mechanically read codes (e.g. a configuration of the absence or presents of cutouts to encode a bit, through which pins or a reader may be inserted).

As used herein, "electrical circuitry" may refer to one or more electrical components, examples of which may include: an Application Specific Integrated Circuit (ASIC) or other programmable logic; electronic/electrical componentry (which may include combinations of transistors, resistors, capacitors, inductors etc); one or more processors (e.g., the circuitry structure of the processor); a non-transitory memory (e.g. implemented by one or more memory devices), that may store one or more software or firmware programs; a combinational logic circuit; interconnection of the aforesaid. The electrical circuitry may be located entirely at the apparatus, or distributed between the apparatus and/or on one or more external devices in communication with the apparatus, e.g. as part of a system. The electrical circuitry may comprise a controller.

As used herein, a "processing resource" (or "processor" or "controller") may refer to one or more units for processing data, examples of which may include an ASIC, microcontroller, FPGA, microprocessor, digital signal processor (DSP) capability, state machine or other suitable component. A processing resource may be configured to execute a computer program, e.g. which may take the form of machine readable instructions, which may be stored on a non-transitory memory and/or programmable logic. The processing resource may have various arrangements corresponding to those discussed for the circuitry, e.g. on-board and/or off board the apparatus as part of the system. As used herein, any machine executable instructions, or computer readable media, may be configured to cause a disclosed method to be carried out, e.g. by a aerosol generating apparatus or system as disclosed herein, and may therefore be used synonymously with the term method.

As used herein, an "external device" (or "peripheral device") may include one or more electronic components external to an aerosol generating apparatus. Those components may be arranged at the same location as the aerosol generating apparatus or remote from the apparatus. An external device may comprise electronic computer devices including: a smartphone; a PDA; a video game controller; a tablet; a laptop; or other like device.

As used herein, a "computer readable medium/media" (or "memory" or "data storage") may include any medium capable of storing a computer program, and may take the form of any conventional non-transitory memory, for example one or more of: random access memory (RAM); a CD; a hard drive; a solid state drive; a memory card; a DVD. The memory may have various arrangements corresponding to those discussed for the circuitry /processor. The present disclosure includes a computer readable medium configured to cause an apparatus or system disclosed herein to perform a method as disclosed herein.

As used herein, a "communication resource" (or "communication interface") may refer to hardware and/or firmware for electronic information/data transfer. The communication resource may be configured for wired communication ("wired communication resources") or wireless communication ("wireless communication resource"). Wireless communication resources may include hardware to transmit and receive signals by radio and may include various protocol implementations e.g. the 802.11 standard described in the Institute of Electronics Engineers (IEEE) and Bluetooth^{™} from the Bluetooth Special Interest Group of Kirkland Wash. Wired communication resources may include; Universal Serial Bus (USB); High-Definition Multimedia Interface (HDMI) or other protocol implementations. The apparatus may include communication resources for wired or wireless communication with an external device.

As used herein, a "network" (or "computer network") may refer to a system for electronic information/data transfer between a plurality of apparatuses/devices. The network may, for example, include one or more networks of any type, which may include: a Public Land Mobile Network (PLMN); a telephone network (e.g. a Public Switched Telephone Network (PSTN) and/or a wireless network); a local area network (LAN); a metropolitan area network (MAN); a wide area network (WAN); an Internet Protocol Multimedia Subsystem (IMS) network; a private network; the Internet; an intranet.

It will be appreciated that any of the disclosed methods (or corresponding apparatuses, programs, data carriers, etc.) may be carried out by either a host or client, depending on the specific implementation (i.e. the disclosed methods/apparatuses are a form of communication(s), and as such, may be carried out from either 'point of view', i.e. in corresponding to each other fashion).

Furthermore, it will be understood that the terms "receiving" and "transmitting" encompass "inputting" and "outputting" and are not limited to an RF context of transmitting and receiving electromagnetic (e.g. radio) waves. Therefore, for example, a chip or other device or component for realizing embodiments could generate data for output to another chip, device or component, or have as an input data from another chip, device, or component, and such an output or input could be referred to as "transmit" and "receive" including gerund forms, that is, "transmitting" and "receiving," as well as such "transmitting" and "receiving" within an RF context.

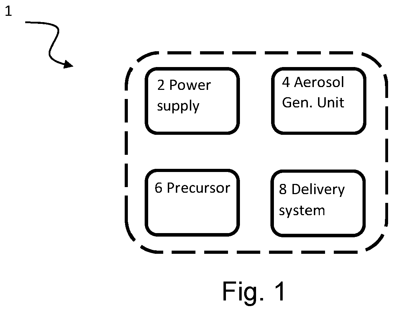

Referring to Fig. 1, an example aerosol generating apparatus 1 includes a power supply 2, for supply of electrical energy. The apparatus 1 includes an aerosol generating unit 4 that is driven by the power supply 2. The power supply 2 may include an electric power supply in the form of a battery and/or an electrical connection to an external power source. The apparatus 1 includes a precursor 6, which in use is aerosolised by the aerosol generating unit 4 to generate an aerosol. The aerosol generating unit 4 includes a piezoelectric transducer (discussed below) configured to induce, by vibration of the piezoelectric transducer i.e. vibration of an aerosolisation surface of the piezoelectric transducer, cavitation in the precursor 6. Collapse of the cavities in the precursor 6 induces a shock that propagates through the liquid precursor 6. This shock disturbs a surface of the liquid precursor 6 that interfaces with air within an aerosolisation chamber of the aerosol generating apparatus 1 (which in turn is in fluid communication with an airflow path within the aerosol generating apparatus). These disturbances take the form of ripples, also known as capillary waves, that form ligaments at the peaks of the ripples/waves, pinch off and expel droplets from the liquid precursor 6 into the airflow path, thereby aerosolising the precursor 6 to generate the aerosol. The apparatus 2 includes a delivery system 8 for delivery of the aerosol to a user.

Electrical circuitry (not shown in figure 1) may be implemented to control the interoperability of the power supply 2 and aerosol generating unit 4.

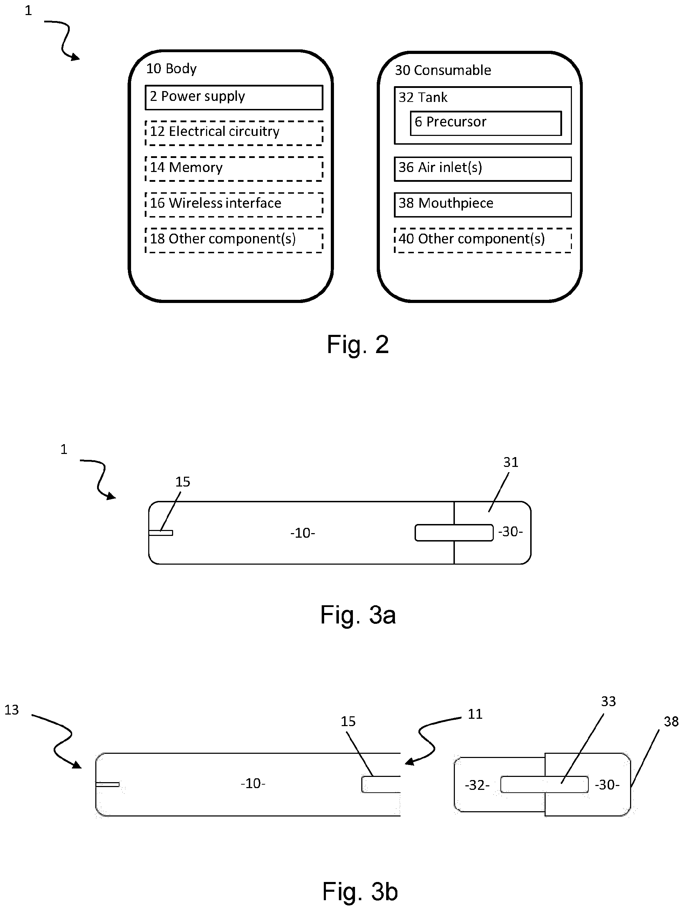

Fig. 2 shows an implementation of the apparatus 1 of Fig. 1, where the aerosol generating apparatus 1 is configured to generate aerosol from a liquid precursor.

In this example, the apparatus 1 includes a device body 10 and a consumable 30.

In this example, the body 10 includes the power supply 2. The body may additionally include any one or more of electrical circuitry 12, a memory 14, a wireless interface 16, one or more other components 18.

The electrical circuitry 12 may include a processing resource for controlling one or more operations of the body 10 and consumable 30, e.g. based on instructions stored in the memory 14.

The wireless interface 16 may be configured to communicate wirelessly with an external (e.g. mobile) device, e.g. via Bluetooth.

The other component(s) 18 may include one or more user interface devices configured to convey information to a user and/or a charging port, for example (see e.g. Fig. 3).

The consumable 30 includes a storage portion implemented here as a tank 32 which stores the liquid precursor 6 (e.g. e-liquid). The consumable 30 also includes one or more air inlets 36, and a mouthpiece 38. The consumable 30 may include one or more other components 40.

The body 10 and consumable 30 may each include a respective electrical interface (not shown) to provide an electrical connection between one or more components of the body 10 with one or more components of the consumable 30. In this way, electrical power can be supplied to components of the consumable 30, without the consumable 30 needing to have its own power supply.

The piezoelectric transducer 34 of the aerosol generating unit 4 is arranged to be in electrical contact with one or more components of the body 10. For example, the power supply 2 may be configured to provide power to the piezoelectric transducer 34. Additionally or alternatively, the piezoelectric transducer 34 may be in electrical contact/communication with one or more of the electrical circuitry 12, memory 14, wireless interface 16 or one or more of the one or more other components 18 e.g., to receive instructions to adjust an operating parameter of the piezoelectric transducer 34 and/or to transmit data indicative of the operational parameters of the piezoelectric transducer 34.

Moreover, the piezoelectric transducer 34 is arranged to be in fluid communication with the tank 32 e.g. via a wick such that the liquid precursor can be provided to the aerosolisation surface of the piezoelectric transducer 34.

In use, a user may activate the aerosol generating apparatus 1 when inhaling through the mouthpiece 38, i.e. when performing a puff. The puff, performed by the user, may initiate a flow through a flow path in the consumable 30 which extends from the air inlet(s) 36 to the mouthpiece 38 via a region (i.e. an aerosolisation chamber) in proximity to the piezoelectric transducer 34.

Activation of the aerosol generating apparatus 1 may be initiated, for example, by an airflow sensor in the body 10 which detects airflow in the aerosol generating apparatus 1 (e.g. caused by a user inhaling through the mouthpiece), or by actuation of an actuator included in the body 10. Upon activation, the electrical circuitry 12 (e.g. under control of the processing resource) may supply electrical energy from the power supply 2 to the piezoelectric transducer 34 of the aerosol generating unit 4, which may cause the piezoelectric transducer 34 to induce cavitation in the liquid precursor 6 drawn from the tank so as to produce an aerosol which is carried by the flow out of the mouthpiece 38.

In some examples, the consumable may include a wick, wherein a first portion of the wick extends into the tank 32 in order to draw liquid precursor 6 out from the tank 32 and wherein a second portion of the wick is arranged to convey the drawn liquid precursor 6 to the aerosolisation surface piezoelectric transducer 34of the aerosol generating unit 4.

In this example, the delivery system 8 is provided by the above-described flow path and mouthpiece 38.

In variant embodiments (not shown), any one or more of the precursor 6, air inlet(s) 36 and mouthpiece 38, may be included in the body 10. For example, the mouthpiece 36 may be included in the body 10 with the precursor 6 arranged as a separable cartomizer.

Figs. 3A and 3B show an example implementation of the aerosol generating apparatus 1 of Fig. 2. In this example, the consumable 30 is implemented as a capsule/pod, which is shown in Fig. 3A as being physically coupled to the body 10, and is shown in Fig. 3B as being decoupled from the body 10.

In this example, the body 10 and the consumable 30 are configured to be physically coupled together by pushing the consumable 30 into an aperture in a top end 11 the body 10, with the consumable 30 being retained in the aperture via an interference fit.

In other examples (not shown), the body 10 and the consumable 30 could be physically coupled together in other ways, e.g. by screwing one onto the other, through a bayonet fitting, or through a snap engagement mechanism, for example.

The body 10 also includes a charging port (not shown) at a bottom end 13 of the body 10.

The body 10 also includes a user interface device configured to convey information to a user. Here, the user interface device is implemented as a light 15, which may e.g. be configured to illuminate when the apparatus 1 is activated. Other user interface devices are possible, e.g. to convey information haptically or audibly to a user.

In this example, the consumable 30 has an opaque cap 31, a translucent tank 32 and a translucent window 33. When the consumable 30 is physically coupled to the body 10 as shown in Fig. 3A, only the cap 31 and window 33 can be seen, with the tank 32 being obscured from view by the body 10. The body 10 includes a slot 15 to accommodate the window 33. The window 33 is configured to allow the amount of liquid precursor 6 in the tank 32 to be visually assessed, even when the consumable 30 is physically coupled to the body 10.

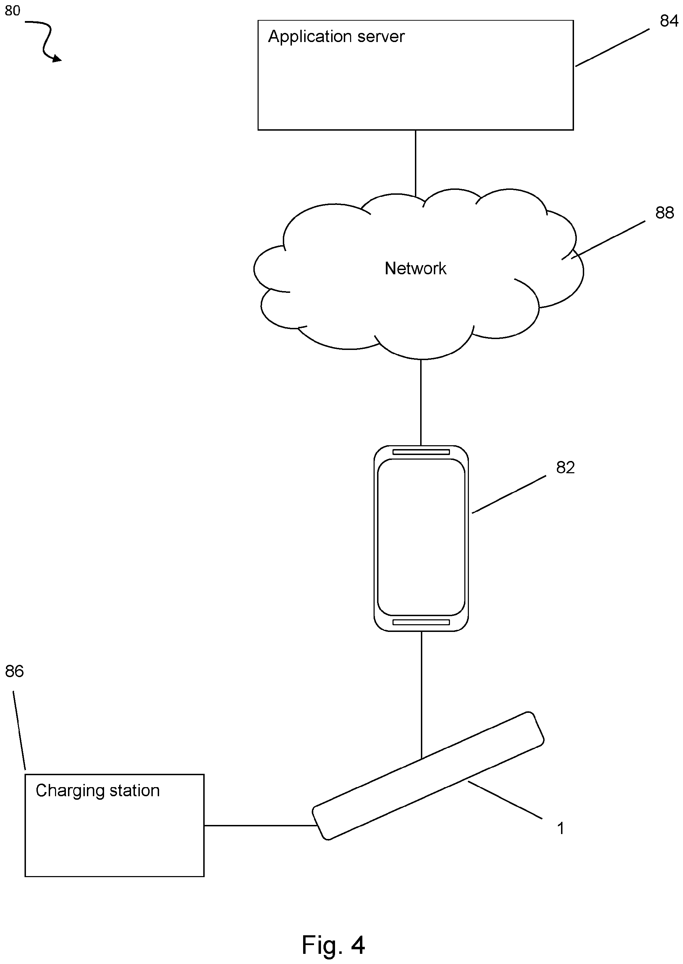

Fig. 4 shows an example system 80 for managing an aerosol generating apparatus 1, such as those described above with reference to any of Figs. 1-3B.

The system 80 as shown in Fig. 1 includes a mobile device 82, an application server 84, an optional charging station 86, as well as the aerosol generating apparatus 1.

In this example, aerosol generating apparatus 1 is configured to communicate wirelessly, e.g. via Bluetooth^{™}, with an application (or "app") installed on the mobile device 2, via a wireless interface included in the aerosol generating apparatus 1 and via a wireless interface included in the mobile device 82. The mobile device 82 may be a mobile phone, for example. The application on the mobile phone is configured to communicate with the application server 84, via a network 88. The application server 84 may utilise cloud storage, for example.

The network 88 may include a cellular network and/or the internet.

In other examples, the aerosol generating apparatus 1 may be configured to communicate with the application server 84 via a connection that does not involve the mobile device 82, e.g. via a narrowband internet of things ("NB-loT") or satellite connection. In some examples, the mobile device 82 may be omitted from the system 80.

A skilled person would readily appreciate that the mobile device 82 may be configured to communicate via the network 88 according to various communication channels, preferably a wireless communication channel such as via a cellular network (e.g. according to a standard protocol, such as 3G or 4G) or via a WiFi network.

The app installed on the mobile device 82 and the application server 84 may be configured to assist a user with managing their aerosol generating apparatus 1, based on information communicated between the aerosol generating apparatus 1 and the app, information communicated directly between the aerosol generating apparatus 1 and the application server 84, and/or information communicated between the app and the application server 84.

The charging station 86 (if present) may be configured to charge (and optionally communicate with) the aerosol generating apparatus 1, via a charging port on the aerosol generating apparatus 1. The charging port on the smoking substitute device 10 may be a USB port, for example, which may allow the aerosol generating apparatus 1 to be charged by any USB-compatible device capable of delivering power to the aerosol generating apparatus 1 via a suitable USB cable (in this case the USB-compatible device would be acting as the charging station 86). Alternatively, the charging station could be a docking station specifically configured to dock with the aerosol generating apparatus 1 and charge the aerosol generating apparatus 1via the charging port on the aerosol generating apparatus 1.

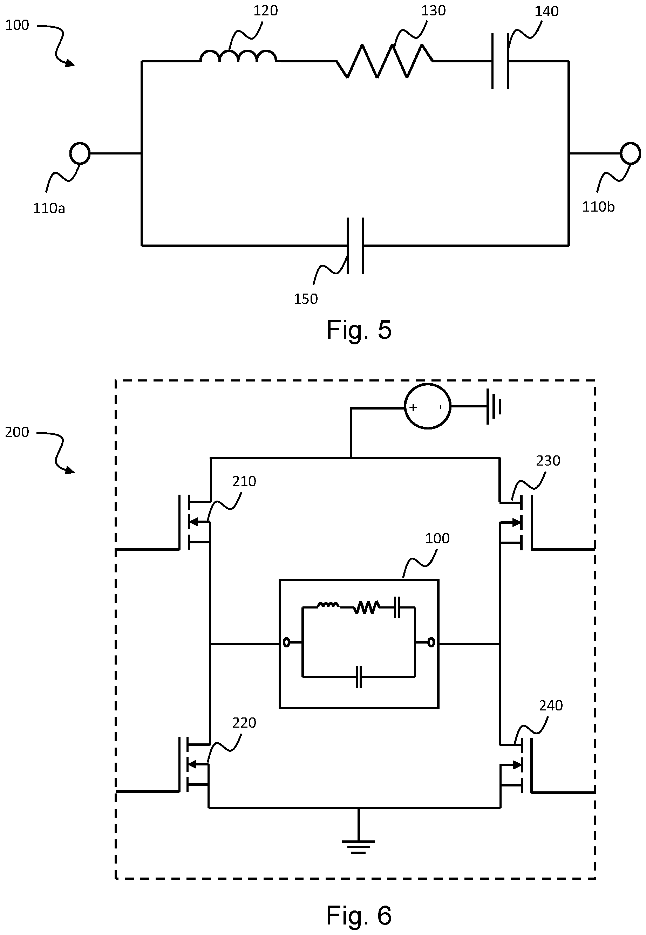

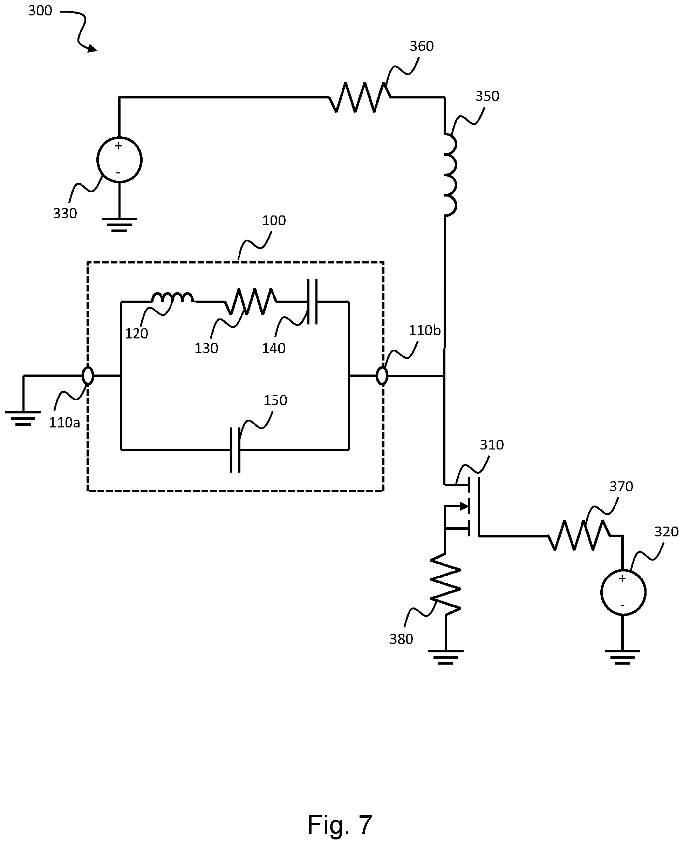

Fig. 5 shows an example of a circuit for modelling the behaviour of a piezoelectric transducer 100 at the resonant frequency of the piezoelectric transducer 100. Example circuits for providing a driving signal to the piezoelectric transducer 100 are described below with reference to Figures 6 and 7.

The circuit includes a set of components connected in series with each other between a pair of terminals 110a, 110b, including: an inductor 120; a resistor 130; and an in-series capacitor 140. The set of series components are connected in parallel with an in-parallel capacitor 150. Each of the components of the circuit model different aspects of the electrical and mechanical behaviour of the piezoelectric transducer 100.

The mechanical vibration of the piezoelectric transducer 100 is modelled by the inductive reactance of the inductor 120, when the frequency of the electric signal driving the piezoelectric transducer 100 is at, or near, the resonant frequency of the piezoelectric transducer 100.

Internal losses associated with the operation of the piezoelectric transducer 100, such as mechanical damping and dielectric losses within the piezoelectric crystal of the transducer 100 are modelled by the resistor 130. The resistance value of the resistor 130 is linked to the quality factor (or Q-factor) of the resonance of the piezoelectric transducer 100, which affects the amplitude and the sharpness of the resonance peak in the transducer's 100 frequency response.

Capacitive mechanical and electrical characteristics of the piezoelectric transducer 100 are modelled by the in-series capacitor 140.

Inherent dielectric properties of the material forming the piezoelectric transducer, e.g., due to the structure of the piezoelectric material between electrodes of the transducer 100 are modelled by the in-parallel capacitor 150. This inherent "parallel" capacitance significantly influences the resonance behaviour of the piezoelectric transducer 100, for example, by affecting the total impedance of the circuit at resonance when combined with the inductive and resistive elements (as modelled by the inductor 120 and the resistor 130).

As an example, the circuit of Figure 5 may be suitable for modelling a typical piezoelectric transducer 100 with a resonance frequency of approximately 3 MHz, by providing the inductor 120 with an inductance of 3 µH, the resistor 130 with a resistance of 4 Ω, the in-series capacitor 140 with a capacitance of 938 pF, and the in-parallel capacitor 150 with a capacitance of 1 nF.

Fig. 6 shows a portion of a conventional driving circuit 200 for driving a piezoelectric transducer 100 using an H-bridge. The H-bridge is defined by four switches 210, 220, 230, 240 arranged in an 'H-shaped' arrangement around the piezoelectric transducer 100. In the example shown in Fig. 6, the four switches 210, 220, 230, 240 are each defined by a respective MOSFET. The H-bridge of Fig. 6 is useful for rapidly changing the polarity of a voltage applied to the piezoelectric transducer 100, thereby driving piezoelectric vibrations in the transducer 100.

H-bridge circuits such as the one depicted in Fig. 6 may face challenges in the context of a user device such as the aerosol-generating apparatus 1 described herein.

For example, high-frequency switching of the four (MOSFET) switches 210, 220, 230, 240 may result in significant power and heat dissipation, generating considerable amounts of heat. This heat can degrade component performance over time and shorten the lifespan of the whole H-bridge, including the piezoelectric transducer 100. Moreover, the power dissipation may represent an undesirable inefficiency in the circuit performance of the H-bridge.

There may also be a risk of a latch-up type short-circuit in which one or more parts of the H-bridge circuit become uncontrollably conductive, thereby compromising the circuit's reliability. In the extreme, latch-up can lead to total circuit failure.

Electromagnetic interference may also be a concern when considering the implementation of a H-bridge. The rapid switching inherent in the operation of the four (MOSFET) switches 210, 220, 230, 240 can generate interference that can disrupt the operation of other electronic components/devices in the vicinity of the H-bridge.

Additionally, the operation of the piezoelectric transducer 100 (or indeed any component having an inductive load), can lead to high-voltage spikes in the current flowing through the circuit. Such spikes risk causing severe damage to the transistors used to embody the four MOSFET switches 210, 220, 230, 240 of the H-bridge shown in Fig. 6.

Furthermore, to induce high-frequency (e.g., ultrasonic) vibrations in the piezoelectric transducer 100, very precise and potentially complex timing control of the H-bridge is required. In particular, if both the first and second switches 210, 220, both the first and third switches 210, 230, both the second and fourth switches 220, 240 or both the third and fourth switches 230, 240 are open at the same time, there is a significant risk of shoot-through, or crossover, current that risks damaging the switches as the shoot-through current passes through and reduces the power efficiency of the H-bridge.

Fig. 7 shows an example of an improved driving circuit 300 for driving a piezoelectric transducer 100 using a single (MOSFET) switch 310.

The driving circuit 300 of Fig. 7 comprises a gate power source 320 configured to controllably apply a voltage to the gate of the MOSFET switch 310 to controllably open and close the MOSFET switch 310. The gate power source 320 may be connected to a clock, or may be an oscillator circuit so as to cyclically open and close the MOSFET switch 310 at a selected frequency.

The driving circuit 300 further comprises a driving power source 330 configured to supply power through the driving circuit 300. When the MOSFET switch 310 is closed, the current supplied by the driving power source 300 bypasses the piezoelectric transducer and flows into the source of the MOSFET switch 310 and out from the drain of the MOSFET switch 310 to ground. The driving power source 330 may be the power supply 2 discussed above in relation to Fig. 1.

When the MOSFET switch 310 is open, the current supplied by the driving power source 300 flows through the piezoelectric transducer 100 to ground, thereby inducing vibration in the piezoelectric transducer.

Application of a current to a piezoelectric transducer 100 induces a mechanical response in the transducer 100. Typically, the current applied to the piezoelectric transducer 100 is an alternating current so as to induce oscillatory vibrations in the piezoelectric transducer 100. Upon application of a current in a first polarity, opposite faces of the piezoelectric crystal of the transducer 100 respond by expanding, or bulging, outwards to define respective convex surfaces. Conversely, upon application of current in a second polarity opposite to the first polarity, the opposite faces of the piezoelectric crystal of the transducer 100 respond by contracting, or drawing, inwards to define respective concave surfaces. In the context of an aerosol-generating apparatus 1, it may be advantageous to only drive the piezoelectric transducer 100 in the first polarity so that physical contact between the transducer 100 and the at least some of the liquid precursor 6 can be maintained. Maintaining this physical contact improves the power efficiency of the inducement of cavitation in the liquid precursor 6, and therefore improves the efficiency of the generation of the aerosol. To this end, the driving signal provided by the driving power source 330 is preferably a direct current power source oscillating, at the piezoelectric transducer 100, between a maximum amplitude and a minimum (zero) amplitude with a frequency corresponding to the switching frequency of the MOSFET switch 310.

The driving circuit 300 may further comprise an inductor 350 connected in series with the piezoelectric transducer. The inductor 350 is arranged and configured with an inductance suitable for smoothing the current profile of the signal provided by the driving power source 330 such that the piezoelectric transducer 100 is not subjected to abrupt step-changes in the voltage and current flowing therethrough. This smoothing of the current profile consequently reduces the risk of damage to the piezoelectric transducer by reducing the risk of harmful voltage spikes.

The driving circuit 300 may further comprise one or more resistors 360, 370, 380 configured to limit the current flowing through the driving circuit.

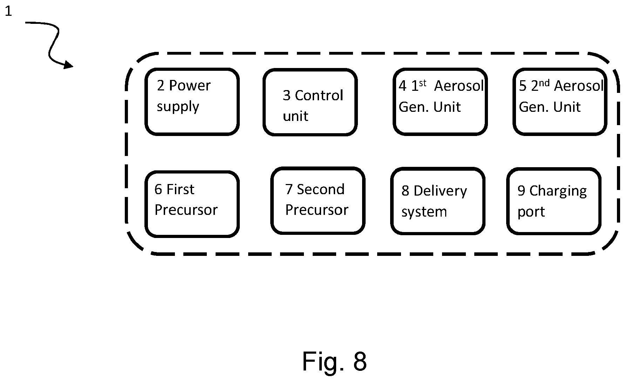

Fig. 8 shows a block system diagram of an example aerosol generating apparatus 1 that includes a power supply 2, for supply of electrical energy. The apparatus 1 includes a first aerosol generating unit 4 that is driven by the power supply 2. The power supply 2 may include an electric power supply in the form of a (rechargeable) battery and/or an electrical connection to an external power source. The apparatus 1 includes a first aerosol precursor 6, which in use is aerosolised by the first aerosol generating unit 4 to generate a first aerosol. The first aerosol generating unit 4 includes a piezoelectric transducer (discussed elsewhere) configured to induce, by vibration of the piezoelectric transducer i.e. vibration of an aerosolisation surface of the piezoelectric transducer, cavitation in the first aerosol precursor 6. Collapse of the cavities in the first aerosol precursor 6 induces a shock that propagates through the first aerosol precursor 6. This shock disturbs a surface of the first aerosol precursor 6 that interfaces with air within an aerosolisation chamber of the aerosol generating apparatus 1 (which in turn is in fluid communication with an airflow path within the aerosol generating apparatus). These disturbances take the form of ripples, also known as capillary waves, that form ligaments at the peaks of the ripples/waves, pinch off and expel droplets from the first aerosol precursor 6 into the airflow path, thereby aerosolising the first aerosol precursor 6 to generate the first aerosol.

The apparatus 1 further includes a second aerosol generation unit 5 that is also driven by the power supply 2. The apparatus further includes a second aerosol precursor 7, which in use is aerosolised by the second aerosol generating unit 5 to generate a second aerosol. The second aerosol generating unit 5 includes a piezoelectric transducer configured to generate second aerosol from the second aerosol precursor 7 in the same manner as the piezoelectric transducer of the first aerosol generation unit 4 is configured to generate the first aerosol from the first aerosol precursor 6.

The apparatus 1 includes a delivery system 8 for delivery of the aerosol to a user.

The apparatus 1 includes a charging port 9 for connecting the power supply 2 to an external power source to charge the power supply 2.

Electrical circuitry (not shown in figure 1) may be implemented to control the interoperability of the power supply 2 and aerosol generating unit 4.

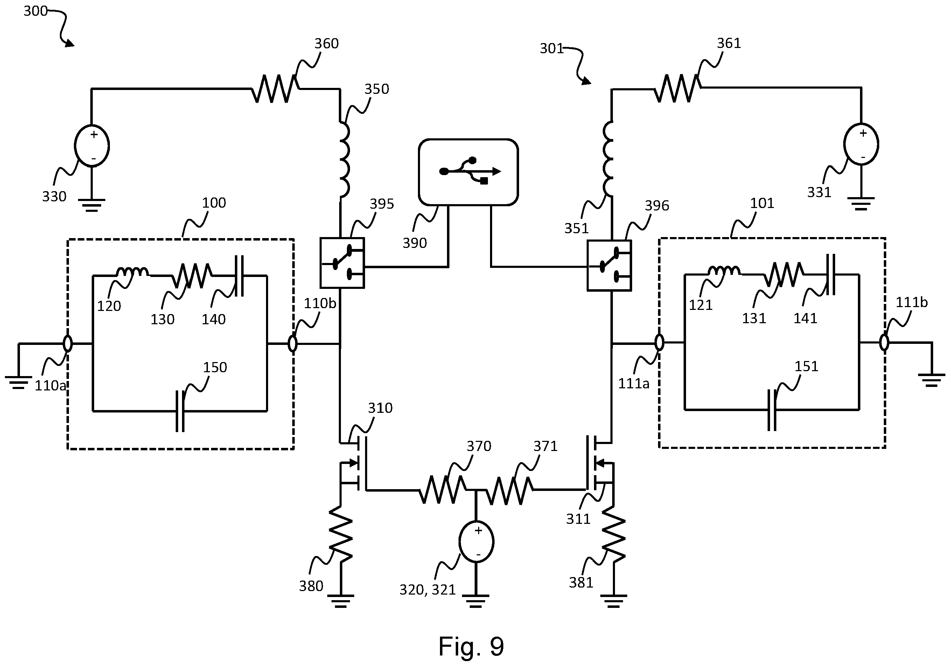

Fig. 9 shows an example of first and second driving circuits 300, 301 for driving respective piezoelectric transducers 100, 101 of the aerosol generating apparatus 1. Each of the first and second driving circuits are, individually, as set out above in relation to Fig. 7.

In other words, the first driving circuit 300 comprises a gate power source 320 operably connected to a MOSFET switch 310. The gate power source 320 may be connected to a clock, or may be an oscillator circuit so as to cyclically open and close the MOSFET switch 310 at a selected (first driving) frequency.

The first driving circuit 300 further comprises a driving power source 330, an inductor 350, and one or more resistors 360, 370, 380.

The first driving circuit 300 further comprises a first three-way switch 395 that is configurable into and between any combination of, or all of: (i) a driving configuration, (ii) a charging configuration, or (iii) an on-charge driving configuration.

When the first three-way switch 395 is in the driving configuration, the first driving circuit 300 is configured such that power is conveyable from the driving power source 330 to the piezoelectric transducer 100.

When the first three-way switch 395 is in the charging configuration, the first driving circuit 300 is configured such that power is conveyable from a charging port 390 to the driving power source 330.

When the first three-way switch 395 is in the on-charge driving configuration, the first driving circuit 300 is configured such that power is conveyable from the charging port 390 to the piezoelectric transducer 100.

The second driving circuit 301 comprises a gate power source 321 operably connected to a MOSFET switch 311. The gate power source 321 may be connected to a clock, or may be an oscillator circuit so as to cyclically open and close the MOSFET switch 311 at a selected (second driving) frequency. As discussed above, in some examples, the first and second gate power source 320, 321 may be either a common gate power source or respectively different power sources.

The second driving circuit 301 further comprises a driving power source 331, an inductor 351, and one or more resistors 361, 371, 381.

The second driving circuit 301 further comprises a second three-way switch 396 that is configurable into any of (i) a driving configuration, (ii) a charging configuration, or (iii) an on-charge driving configuration.

When the second three-way switch 396 is in the driving configuration, the second driving circuit 301 is configured such that power is conveyable from the driving power source 331 to the piezoelectric transducer 101.

When the second three-way switch 396 is in the charging configuration, the second driving circuit 301 is configured such that power is conveyable from the charging port 390 to the driving power source 331.

When the second three-way switch 396 is in the on-charge driving configuration, the second driving circuit 301 is configured such that power is conveyable from the charging port 390 to the piezoelectric transducer 101.

In some implementations, when one of the first and second three-way switches 395, 396 is in the charging configuration, the other may be in the on-charge driving configuration.

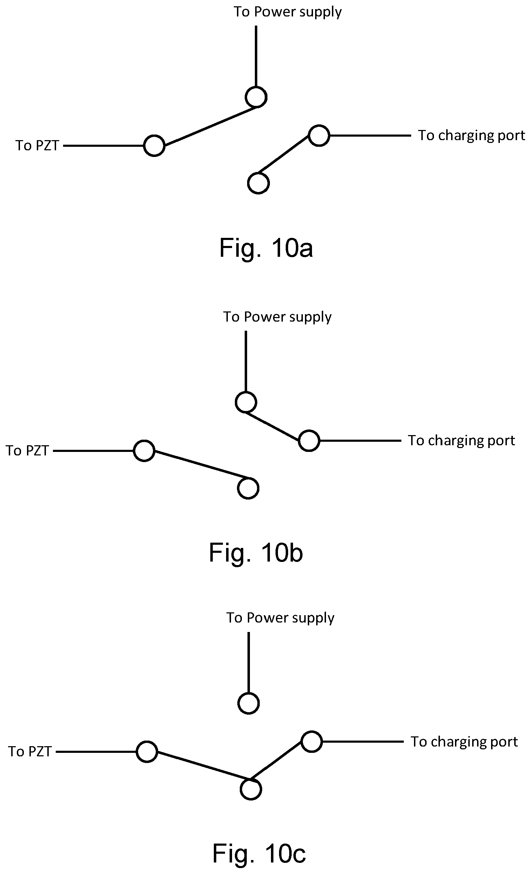

Figs. 10a to 10c show exemplary configurations of the first and second three-way switches 395, 396 suitable for switching the driving circuits 300, 301 of Fig. 9 between the driving configuration (Fig. 10a), the charging configuration (Fig. 10b) and the on-charge driving configuration (Fig. 10c).

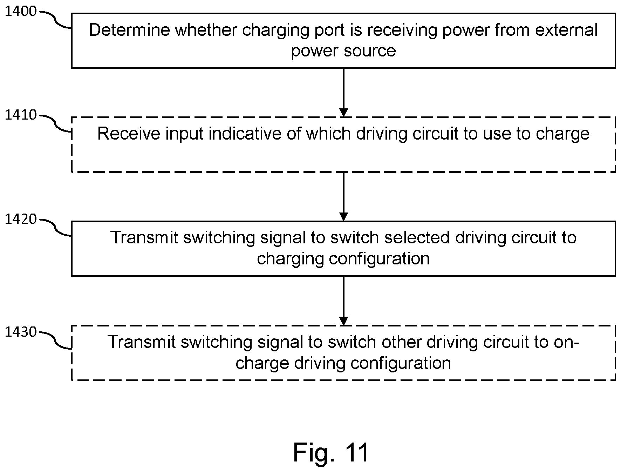

Fig. 11 shows an exemplary method of controlling an aerosol generating apparatus.

The method comprises, in an operation 1400, determining whether the charging port 9, 390 is receiving power from an external power source.

The method may further comprise, in an operation 1410, receiving input (e.g., from a user via a user interface communicatively connected to a communications interface of the aerosol generating apparatus 1) indicative of which of the first and second driving circuits 300, 301 should be switched to the charging configuration.

The method further comprises, in an operation 1420, transmitting a first switching signal to switch one of the first and second driving circuits 300, 301 (e.g., the driving circuit selected in operation 1410) to the charging configuration.

The method may further comprise, in an operation 1430, transmitting a second switching signal to switch the other of the first and second driving circuits 300, 301 (e.g., the non-selected driving circuit) to the on-charge driving configuration.

### REFERENCES

The entirety of the following documents, which are referenced in the present disclosure, are incorporated by reference into the present disclosure:
- Kooij, S., Astefanei, A., Corthals, G.L. et al. Size distributions of droplets produced by ultrasonic nebulizers. Sci Rep 9, 6128 (2019). https://doi.org/10.1038/s41598-019-42599-8

## Claims

1. An aerosol generating apparatus (1) comprising:
a first aerosol generation unit (4) configured to generate a first aerosol from a first aerosol precursor (6);
a second aerosol generation unit (5) configured to generate a second aerosol from a second aerosol precursor (7);
a power supply (2) arranged to provide power to the first and second aerosol generation units (4, 5);
a charging port (9) arranged to receive power from an external power source so as to charge the power supply (2);
a first driving circuit configured, in a driving configuration, to drive the first aerosol generation unit (4) to generate the first aerosol; and
a second driving circuit configured, in a driving configuration, to drive the second aerosol generation unit (5) to generate the second aerosol,
wherein the first and/or second driving circuits are controllably switchable to switch to a charging configuration,
wherein, when the first or second driving circuit is in the charging configuration, said driving circuit is configured to transfer power from the charging port (9) to the power supply (2) to charge the power supply.

2. The aerosol generating apparatus (1) according to claim 1, wherein the first and/or second driving circuits are further controllably switchable to switch from the driving configuration to an on-charge driving configuration in response to a determination that the charging port (9) is receiving power from an external power source, such that:
in response to the determination that the charging port (9) is receiving power from an external power source, one of the first and second driving circuits is switched to the charging configuration, and the other of the first and second driving circuits is switched to the on-charge driving configuration,
wherein, when the first or second driving circuit is in the on-charge driving configuration, said driving circuit is configured to transfer power from the charging port (9) to the corresponding aerosol generation unit (4,5) so as to generate a corresponding aerosol.

3. The aerosol generating apparatus (1) according to claim 1 or 2, wherein the first aerosol generation unit (4) is a first piezoelectric transducer (100) and/or the second aerosol generation unit (5) is a second piezoelectric transducer (101).

4. The aerosol generating apparatus (1) according to claim 3, wherein the first driving circuit includes a first switch (310) for facilitating the application of current at a first driving frequency through the first piezoelectric transducer (100) so as to induce vibrations in the first piezoelectric transducer at the first driving frequency, and/or
wherein the second driving circuit driving circuit includes a second switch (311) for facilitating the application of current at a second driving frequency through the second piezoelectric transducer (101) so as to induce vibrations in the second piezoelectric transducer at the second driving frequency.

5. The aerosol generating apparatus according to claim 4, wherein the current respectively applied through the first and/or second piezoelectric transducer (100, 101) is a direct current such that the corresponding piezoelectric transducer is driven with a current having a single polarity.

6. The aerosol generating apparatus according to claim 4 or 5, wherein the first switch (310) and/or the second switch (311) is a MOSFET switch driven by a common or respective MOSFET power source (320, 321).

7. The aerosol generating apparatus according to any of claims 4 to 6, wherein the first switch is connected to an oscillator or clock for actuating the switch at the first driving frequency, and/or the second switch is connected to a same or different oscillator or clock for actuating the switch at the second driving frequency.

8. The aerosol generating apparatus according to any preceding claim, wherein the power supply (2) is a rechargeable battery.

9. The aerosol generating apparatus according to any preceding claim wherein the first aerosol precursor is different from the second aerosol precursor.

10. The aerosol generating apparatus according to claim 9, wherein the first aerosol precursor includes a nicotine-containing component, and/or the second aerosol precursor includes a flavouring.

11. A method of controlling an aerosol generating apparatus according to any preceding claim, the method comprising:
determining whether the charging port (9) is receiving power from an external power source; and
in response to a determination that the charging port (9) is receiving power from an external power source, transmitting a switching signal to the first driving circuit or the second driving circuit to cause said driving circuit to switch to the charging configuration.

12. The method according to claim 11, the method further comprising:
receiving input indicative of a user selection of which of the first and second driving circuits to switch to the charging configuration; and
transmitting the switching signal to the selected driving circuit.

13. The method according to claim 11 or 12, wherein the aerosol generating apparatus is as set out in claim 2 or any claim dependent thereon, the switching signal is a first switching signal, and the method further comprises:
transmitting a second switching signal to the other of the first and second driving circuits to cause said other driving circuit to switch to the on-charge driving configuration.

14. A computer-readable medium comprising instructions that, when executed by a computer or electrical circuitry, cause the computer or electrical circuitry to carry out the method of any of claims 11 to 13.

15. Electrical circuitry for an aerosol generating system, the electrical circuitry being arranged to perform the method of any of claims 11 to 13.
